# EUROPÄISCHE PATENTSCHRIFT

(11) **EP 1 893 136 B1**
(45) Veröffentlichungstag und Bekanntmachung des Hinweises auf die Patenterteilung: **26.10.2011**
(21) Anmeldenummer: 06754460.1
(22) Anmeldetag: 20.06.2006
(51) Int. Cl.: A61F 2/44

(54) **ZWISCHENWIRBELPROTHESE MIT SELBSTSCHNEIDENDEN FIXIERVORSPRÜNGEN**
INTERVERTEBRAL PROSTHESIS COMPRISING SELF-TAPPING FIXING PROJECTIONS
PROTHESE INTERVERTEBRALE POURVUE DE STRUCTURES SAILLANTES DE FIXATION AUTOTARAUDEUSES

(30) Priorität: 22.06.2005 EP 05013517
(43) Veröffentlichungstag der Anmeldung: 05.03.2008
(73) Patentinhaber: Cervitech, Inc., San Diego CA 92121 (US)
(72) Erfinder: KELLER, Arnold, 23863 Kayhude (DE)
(74) Vertreter: Peterreins, Frank
(86) Internationale Anmeldenummer: PCT/EP2006/005910
(87) Internationale Veröffentlichungsnummer: WO 2006/136373

(56) Entgegenhaltungen:
- EP-A- 1 405 615
- WO-A-00/40179
- WO-A-2005/007040
- US-A1- 2003 069 640
- US-A1- 2004 002 761

## Beschreibung

Zwischenwirbelprothesen bedürfen einer Fixierung an den benachbarten Wirbelkörpern, damit sie die ihnen zugedachte Stellung nicht verlassen. Es ist bekannt, sie mit Rippen oder Zapfen zu versehen, die in formentsprechende Fixiervertiefungen der wirbelkörper-Deckplatten eingesetzt werden (WO 01/01893, WO 2004/080355, DE-3023353, FR-A-2659226). Für deren Herstellung ist eine tiefgreifende Bearbeitung der Wirbelkörper-Deckplatten erforderlich, die einen beträchtlichen Operationsaufwand bedeutet. Unerwünscht und im Bereich der Halswirbelsäule oft unmöglich ist eine solche Bearbeitung der Wirbelkörper-Deckplatten auch, weil sie eine starke Distraktion verlangt und eine beträchtliche Dicke der Wirbelkörper voraussetzt. Insbesondere für die Halswirbelsäule werden deshalb Prothesenbauarten bevorzugt, die keine Ausarbeitung von Fixiervertiefungen in den Deckplatten der Wirbelkörper verlangen. Zu diesen gehören solche Prothesen, bei denen die Außenflächen der Anschlußplatten eine im wesentlichen vollflächige, quer verlaufende Zahnung mit Sägezahnprofil aufweisen, dessen steile Flanken ventral angeordnet sind (WO 2004/089259, FR-A-2718635). Damit kann im Normalfall eine Verschiebung der Prothese nach ventral verhindert werden, jedoch gibt es Fälle, in denen sich der Zwischenwirbelraum aufgrund ungewöhnlicher Anatomie oder ungeeigneter chirurgischer Vorbereitung so stark nach ventral keilförmig öffnet, daß die Prothese allein durch die Zahnung nicht mit Sicherheit gehalten werden kann. Das liegt daran, daß die Zahnung nicht in die Knochenoberfläche eindringt. Das soll sie nicht, um die Knochenrinde nicht großflächig zu verletzen und dadurch zu schwächen. Sie soll vielmehr auf der Knochenoberfläche aufliegen, was im Normalfäll zur Fixierung hinreicht, da sie sich auch dann an Unebenheiten festhakt. Bekannt sind ferner Zwischenwirbelprothesen mit selbstschneidenden Fixiervorsprüngen (EP-A-1057462, EP-A-1103237). Die Außenfläche der Anschlußplatten der Prothese bildet eine Anschlußfläche für die Verbindung mit dem Wirbelkörper. Sie umfaßt eine Grundfläche, die dazu bestimmt ist, an der Knochenoberfläche anzuliegen, und davon sich erhebende Fixiervorsprünge, die so scharf ausgebildet sind, daß sie unter der natürlichen Belastung des Gelenks durch die Kraft der Bänder und das zu übertragende Gewicht in die Knochenoberfläche eindringen, sobald die Distraktion der Wirbelkörper aufgehoben ist. Allerdings verlangen die bekannten Prothesen dieser Art eine beträchtliche Distraktion, damit sie unbehindert von den Fixiervorsprüngen eingebracht werden können. Diese kann im Bereich der Lenden- und Brustwirbelsäule ggf. toleriert werden, nicht aber im Bereich der Halswirbelsäule. Würde man sie ohne hinreichende Distraktion einpressen, so würden die Fixiervorsprünge Rinnen in den Wirbelkörper-Deckplatten hobeln, durch die sie anschließend wieder herausgleiten können und die auch wegen der Festigkeitseinbuße unerwünscht sind. Letzteres gilt auch für die in der Implantationsrichtung verlaufenden Rippen einer weiteren bekannten Prothese (WO 2005/007040, Fig. 5 bis 8), wenn diese unterbrochen sind und im Bereich der Unterbrechung steil nach ventral abfallende Stirnflächen bilden. Schließlich ist eine Prothese bekannt (US 2004/0002761 A, Fig 26A und 26B), deren Anschlußflächen am Rand eine Reihe von radial gerichteten Fixiervorsprüngen tragen, von denen jeder auf der Basis eines in Radialrichtung langgestreckten gleichseitigen Dreiecks symmetrisch beiderseits abfallende Flanken aufweist. Je zwei Fixiervorsprünge auf jeder Seite verlaufen gegensinnig schräg zur Medianebene. Da ihre in der Implantationsrichtung weisenden Seitenflächen nicht weniger steil als ihre entgegen der Implantationsrichtung weisenden Seitenflächen verlaufen, haben sie den oben erwähnten Nachteil, daß sie entweder eine beträchtliche Distraktion des Zwischenwirbelraums für die Implantation verlangen oder, wenn man auf diese verzichtet, Rinnen in die Knochenoberfläche schneiden, durch die sie anschließend wieder herausgleiten können.

Das der Erfindung zugrundeliegende Problem besteht daher darin, eine Zwischenwirbelprothese mit selbstschneidenden Fixiervorsprüngen zu schaffen, die auch bei geringer Distraktion der Wirbelkörper in eine sichere Fixierstellung gebracht werden können, ohne die Wirbelkörperdeckplatten ventral von der Stelle ihrer Fixierposition übermäßig zu verletzen.

Ausgehend von dem letztgenannten Stand der Technik besteht die erfindungsgemäße Lösung in den Merkmalen des Anspruchs 1. Demnach werden die Fixiervorsprünge von wenigstens einem Paar von gegensinnig schräg zu der Implantantionsrichtung verlaufenden Rippen gebildet. Die Implantationsrichtung ist im allgemeinen die AP-Richtung (AP = anterior-posterior = von vorne nach hinten). Zweckmäßigerweise sind die Rippen symmetrisch zur Medianebene (mittlere Sagittalebene) angeordnet. Infolge ihrer Schrägstellung haben die Rippen eine mehr und eine weniger entgegen der Implantationsrichtung gewendete Seitenfläche. Die mehr entgegen und i.a. nach vorne gewendete Seitenfläche ist steil und vorzugsweise etwa lotrecht zu der Grundfläche ausgebildet, um eine erheblichen widerstand gegen eine nach vorne gerichtet Bewegung der Prothese zu erzeugen und sie dadurch im Zwischenwirbelraum festzuhalten. Die andere Seitenfläche, die weniger entgegen der Implantationsrichtung und im allgemeinen nach dorsal gewendet ist, ist sanft geneigt, um beim Einsetzen der Prothese den Widerstand herabzusetzen und die Knochenoberfläche zu schonen, über die die Rippen hinweggleiten. Dank ihrer geringeren Neigung bildet sie eine Aufgleitfläche. Etwaige Unebenheiten der Knochenoberfläche, die bei steiler und scharfer Ausbildung der bei der Implantation vorangehenden Seite der Rippen abgehobelt würden, können über diese Fläche auf die Rippe ohne oder mit geringerer Beschädigung aufgleiten. Sie soll, gemessen in einer parallel zur Implantationsrichtung verlaufenden Ebene, einen Winkel von weniger als 45° mit der Grundfläche einschließen. Dabei wird vorausgesetzt, daß die Grundfläche etwa parallel zu den Hauprichtungen der Anschlußplatte verläuft. Die Aufgleitfläche kann glatt ausgeführt werden, um wenig Widerstand zu bieten und die Knochenoberfläche möglichst nicht zu verletzen. Jedoch ist auch eine Ausführung von Vorteil, bei welcher die Aufgleitfläche rauh ausgebildet und beispielsweise mit kleinen Zähnen versehen ist, die sich nach der Implantation innig mit der Knochenoberfläche verbinden, um eine zusätzliche Fixierwirkung zu erzielen.

Die Rippe kann ein im wesentlichen gleichmäßig durchgehendes Profil aufweisen. Dies Profil soll hinreichend scharf sein, damit die Rippe unter der Gelenkbelastung nach Aufhebung der Distraktion in die Knochenoberfläche eindringen kann. Die Aufgleitfläche wird dann von ihrer mehr nach dorsal gewendeten Seitenfläche gebildet.

Bevorzugt wird eine Ausführung, bei der die Rippe unter Bildung einer Reihe von spitzen oder scharfen Zähnen mehrfach durchbrochen ist. Dadurch wird die Größe der Querschnittsfläche der Rippe (in einer parallel zur Flächenerstreckung der Prothese verlaufenden Schnittebene) vermindert, die den Widerstand gegen das Eindringen in die Knochenoberfläche bestimmt. Diese von Zähnen gebildete Rippe dringt daher leichter und tiefer in die Knochenoberfläche ein als eine durchgehende Rippe ähnlicher Profilform. Wenn die Durchbrechungen nach einem weiteren Erfindungsmerkmal quer zur AP-Richtung verlaufen, bilden sie zusätzliche Flächen, die einer Bewegung der Prothese nach ventral entgegenwirken. Auch diese Flächen sollen deshalb steil sein. Die Durchbrechungen lassen sich leichter herstellen, wenn sie in allen Rippen parallel verlaufen.

Die Zähne, die in dieser Ausführungsform die Rippen bilden, weisen vier Begrenzungsflächen auf. Eine erste Begrenzungsfäche wird von der schräg zur Sagittalrichtung verlaufenden und mehr nach ventral gewendeten Seitenfläche der Rippe gebildet. Eine zweite Begrenzungsfläche ist die der ersten gegenüberliegende Seitenfläche der Rippe, die in der Regel parallel zu der ersten verläuft und mehr in Implantationsrichtung gewendet ist. Eine dritte und eine vierte Begrenzungsfläche werden von den Durchbrechungen zwischen den Zähnen gebildet. Die dritte Fläche ist entgegen der Implantationsrichtung gewendet und ist steil. Die vierte ist in Implantationsrichtung gewendet und weniger steil. Die dritte und vierte Fläche geben den Zähnen ein Sägezahnprofil. Sowohl die zweite als auch Die vierte Fläche wirkt als Aufgleitfläche. Auch die zweite Fläche kann als Aufgleitfläche zur Prothesenebene geneigt sein, wobei aber die vierte Fläche meist die für den Aufgleitwiderstand wichtigere ist.

Bei einer anderen Ausführungsform der Erfindung verlaufen die Durchbrechungen parallel zur Implantationsrichtung. Die Größe der auf die Knochenoberfläche bei der Implantation schabend einwirkenden Kante wird dadurch vermindert. In diesem Fall bilden nur die schräg zur Implantationsrichtung verlaufenden Seitenflächen der Rippe auf der ventralen Seite den Widerstand gegen die Bewegung der Prothese nach ventral und auf der dorsalen Seite die Aufgleitfläche.

Wenn im vorliegenden Zusammenhang der Begriff steil verwendet wird, so ist damit ein Winkel zur Grundfläche gemeint, der nahe bei 90° liegt. Er ist zweckmäßigerweise größer als 70°, wobei dieser Winkel entweder in einer lotrecht zu der gemessenen Flanke und der Grundfläche liegenden Ebene oder vorzugsweise in einer Sagittalebene gemessen wird. Er kann über 90° hinausgehen (Hinterschnitt).

Die selbstschneidene Eigenschaft der Rippen wird hauptsächlich durch ihre Querschnittsfläche parallel zur Grundfläche beschrieben. Damit die Gelenkkräfte ausreichen, die Rippen in die Knochenoberflächer hinreichend einzudrücken, nehmen diese nur einen kleinen Teil der gesamten Anschlußfläche ein. Der Flächenanteil an der Anschlußfläche, den sie bei vollständiger Einbettung in den Knochen einnehmen - das ist ihr gesamter Flächenanteil an der Anschlußfläche - soll nicht größer als ein Fünftel, vorzugsweise nicht größer als ein Zehntel sein. Da es nicht erforderlich ist, daß sie sich schon unmittelbar nach der Operation vollständig in den Knochen eingedrückt haben, ist es zweckmäßig, wenn ihre Querschnittsfläche parallel zur Grundfläche in mittlerer Höhe nicht größer ist als ein Zehntel, vorzugsweise ein Zwanzigstel der Anschlußfläche. Sie sollen scharf bzw. spitz sein, damit sie sich auch im Fall ungewöhnlich geringer Gelenkkräfte unmittelbar postoperativ ein wenig eindrücken. Die Begriffe scharf oder spitz bedeuten, daß der Schneiden- bzw. Spitzenwinkel nicht über 50° liegt. Bei einer Spitze, die in unterschiedlichen Ebenen verschiedene Spitzenwinkel zeigt, ist der kleinste Spitzenwinkel maßgebend.

Die Endplatten der Wirbelkörper setzen dem Eindrücken der Rippen bzw. der diese bildenden Zähne um so mehr Widerstand entgegen, je dichter sie sind. Da die Dichte im mittleren Bereich geriner ist als außen, sollen die Rippen hauptsächlich im inneren Bereich der Anschlußflächen angeordnet sein. Genauer gesagt, sollen sie mindestens mit der Hälfte der von ihnen eingenommenen Fläche einen Abstand von mehr als einem Sechstel der AP-Dimension der Anschlußfläche von deren nächstgelegener Begrenzung haben. Insbesondere sollen die dorsolateralen Bereiche der Anschlußfläche von den Rippen frei sein, damit die Grundfläche dort auf dichtes, unverletztes Knochenmaterial Kraft übertragen kann.

Vorzugsweise sind die Rippen V-förmig mit nach vorne sich öffnendem Zwischenraum angeordnet. Wenigstens die Rippen eines Rippenpaars nähern sich einander nach dorsal. Zwar ist auch eine umgekehrte Anordnung denkbar. Jedoch hat es sich bewährt, wenn die Rippen zwischen sich auf ihrer ventralen Seite einen zusammenhängenden Block von Knochenmaterial einschließen, der sie an einer Bewegung nach ventral hindert. Es können auf jeder Seite der Symmetrieachse mehrere parallele Rippen angeordnet sein. Im allgemeinen genügt jedoch eine Rippe auf jeder Seite.

Die Höhe der Rippen über der Grundfläche soll ausreichend sein für einen sicheren Eingriff im Knochenmaterial. Sie soll in der Regel 0,5 mm nicht unterschreiten. Ihre Höhe ist durch den Wunsch begrenzt, bei der Implantation die Distraktion und die Abschabung der Wirbelkörper-Deckplatten durch die Fixiervorsprünge gering zu halten. Sie soll in der Regel 2 (vorzugsweise 1,6) mm oder ein Achtel (vorzugsweise eine zehntel) der AP-Abmessung der Anschlußfläche nicht überschreiten. Bewährt hat sich für Zervikalprothesen eine Höhe von 0,7 bis 1,5 mm. Die Höhe ist über der Grundfläche zu messen, d.h. derjenigen die Rippen umgebenden Fläche, von der zu erwarten ist, daß sie sich am Knochen anlegt. Wenn diese Fläche eine Rauhigkeit aufweist, beispielsweise eine poröse Beschichtung oder eine im wesentlichen vollflächige Ausrüstung mit Zahnreihen, in deren Zwischenräume im Laufe der Zeit Knochensubstanz einwachsen soll, so ist die Oberfläche dieser Rauhigkeit maßgebend.

Der Winkel, den die Rippen mit der Implantationsrichtung einschließen, braucht nicht groß zu sein. Vorzugsweise ist der Winkel, den die mehr nach ventral gewendete Seitenflächen der Rippen miteinander bilden, kleiner als 60°, weiter vorzugsweise kleiner als 30°. Besonders bewährt haben sich Winkel zwischen etwa 10 und 20°.

Der dauerhafte Sitz der Prothese ist besonders dann gefährdet, wenn sich der Wirbelzwischenraum nach ventral stark keilförmig öffnet. Da in diesem Fall die Verbindung zwischen den Anschlußplatten der Prothese und den Endplatten der Wirbelkörper im dorsalen Bereich fester als im ventralen Bereich ist, sieht die Erfindung vor, daß die Rippen mindestens auch im dorsalen Bereich der Anschlußflächen wirksam sind. Zu diesem Zweck soll mindestens ein Rippenpaar bis wenigstens auf ein Fünftel der AP-Abmessung der Anschlußfläche an deren dorsale Begrenzung heranreichen, vozugsweise bis mindestens bis auf ein Zehntel.

Die Erfindung wird im folgenden näher unter Bezugnahme auf die Zeichnung erläutert, die vorteilhafte Ausführungsbeispiele veranschaulicht. Es zeigen:
- Fig. 1: eine Draufsicht auf eine Anschlußplatte,
- Fig. 2: einen Schnitt gemäß der Linie II-II der Fig. 1
- Fig. 3: einen Schnitt gemäß der Linie III-III der Fig. 1,
- Fig. 4: eine Draufsicht auf eine zweite Ausführungsform,
- Fig. 5: eine Ansicht gemäß Pfeil V der Fig. 4,
- Fig. 6: dieselbe Ansicht einer geänderten Ausführung,
- Fig. 7: einen Schnitt gemäß Linie VII-VII der Fig. 4 und
- Fig. 8 und 9: Draufsichten auf weitere Rippenanordnungen.

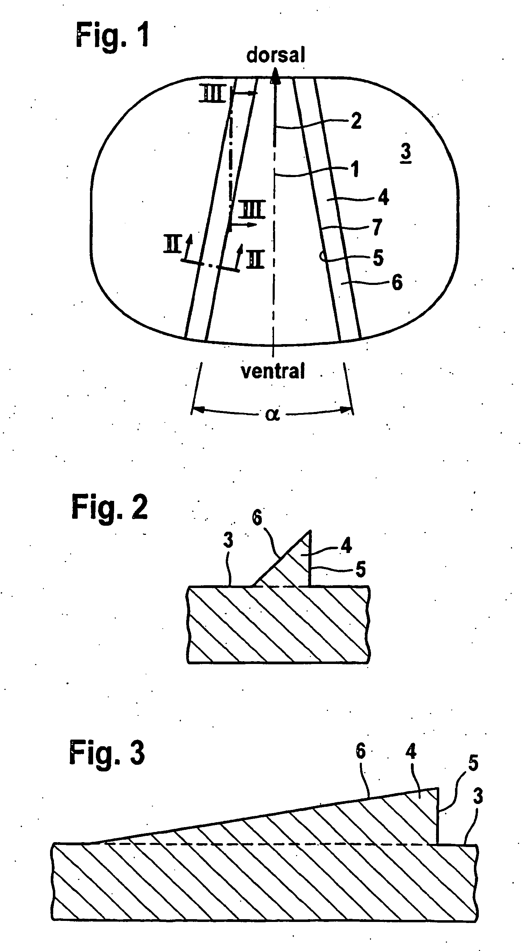

Fig. 1 zeigt den Blick auf die Anschlußfläche einer Anschlußplatte der Prothese, die symmetrisch zu der Medianebene 1 ausgebildet ist. Ihre Anpassung an die Form des Wirbelzwischenraums bringt es mit sich, daß sie eine vorbestimmte Ventralseite und Dorsalseite hat. Ebenso ist die Implantationsrichtung vorherbestimmt, die im Fall der abgebildeten Beispiele die von ventral nach dorsal führende Richtung 2 ist.

Die Anschlußfläche enthält eine Grundfläche 3, die sich möglichst vollflächig an den Knochen anlegen soll und mit eine Oberflächenstruktur versehen sein kann, die eine innige Verbindung durch in Poren oder andere vertiefungen einwachsendes Knochengewebe ermöglicht. Die Grundfläche umgibt zwei Rippen 4, die symmetrisch zur Medianebene 1 angeordnet sind und miteinander einen Winkel alpha von etwa 20° einschließen. Ihre mehr nach ventral gewendete Seitenfläche 5 ist steil, nämlich etwa 90° zur Grundfläche 3 angeordnet, und die dazu parallel verlaufende, mehr nach dorsal gewendete Seitenfläche 6 ist in einer lotrecht zur Rippe verlaufenden Schnittebene (Fig. 2) schräg unter etwa 45° zur Grundfläche 3 geneigt. In einem parallel zur Implantationsrichtung verlaufenden Schnitt (Fig. 3) erscheint der Winkel wesentlich flacher. Jedenfalls ist sie so angeordnet, daß sie auf die Knochen- oder Knorpelfläche der ihr zugeordneten Wirbelkörperdeckplatte bei der Implantationsbewegung sanft aufgleitet. Das gilt auch dann, wenn die beteiligten Wirbelkörper zuvor nicht auf einen Abstand distrahiert wurden, der größer als die Dicke der Prothese ist. Deshalb wird sie als Aufgleitfläche bezeichnet. Da die beiden Seitenflächen 5 und 6 miteinander einen Winkel von etwa 45° (oder vorzugsweise noch weniger) einschließen, bilden sie am Kopf der Rippe eine scharfe Schneide 7. Wenn die Kraft der die Wirbelkörper verbindenden Bänder und die von der Wirbelsäule aufzunehmende Last die beteiligten Wirbelkörper nach der Implantation einander nähern, dringt die Rippe ganz oder teilweise in das Knochengewebe ein und verankert dadurch die Prothese in der gewünschten Stellung. Es ist nicht erforderlich, daß sie sich sofort nach der Operation mit ihrer vollen Höhe in den Knochen einsenkt; jedoch wird angestrebt, daß sich die Grundfläche 3 innerhalb kurzer Zeit nach der Operation großflächig an die Knochenoberfläche anlegt. Dies wird einerseits durch die Schärfe der Rippe und andererseits durch ihren geringen Flächenanteil an der Gesamtfläche der Anschlußfläche erreicht. Dieser Flächenanteil ist am Kopf der Rippe minimal und steigt bis zu ihrer mittleren Höhe auf etwa 5% der Größe der Anschlußfläche an. Es ist zu erwarten, daß sich die Rippe kurz nach der Operation mindestens etwa so tief in den Knochen einsenkt. Ihr an ihrem Fuß gemessener Flächenanteil ist etwa doppelt so groß.

Die fixierende Wirkung der Rippen 3 beruht auf ihrem Zusammenspiel mit dem zwischen ihnen eingeschlossenen Knochenmaterial. Die Prothese könnte dem Wirbelzwischenraum nur dann entweichen, wenn ihre mehr nach ventral gewendeten Seitenflächen 5 dieses Knochenmaterial zwischen sich komprimieren würden. Es setzt dieser Verformung einen so großen Widerstand entgegen, daß das Implantat dadurch in seiner Position hinreichend gesichert ist. Das gilt auch unmittelbar nach der Operation, weil durch die geneigte Form der Aufgleitfläche 6 bewirkt wird, daß das Knochen- oder Knorpelmaterial, das jeder Rippe ventral bzw. auf der Innenseite unmittelbar benachbart ist, während der Implantation nicht oder nur wenig durch die Rippe 4 abgefräst oder abgeschabt wurde und somit für die Halterung der Prothese noch zur Verfügung steht.

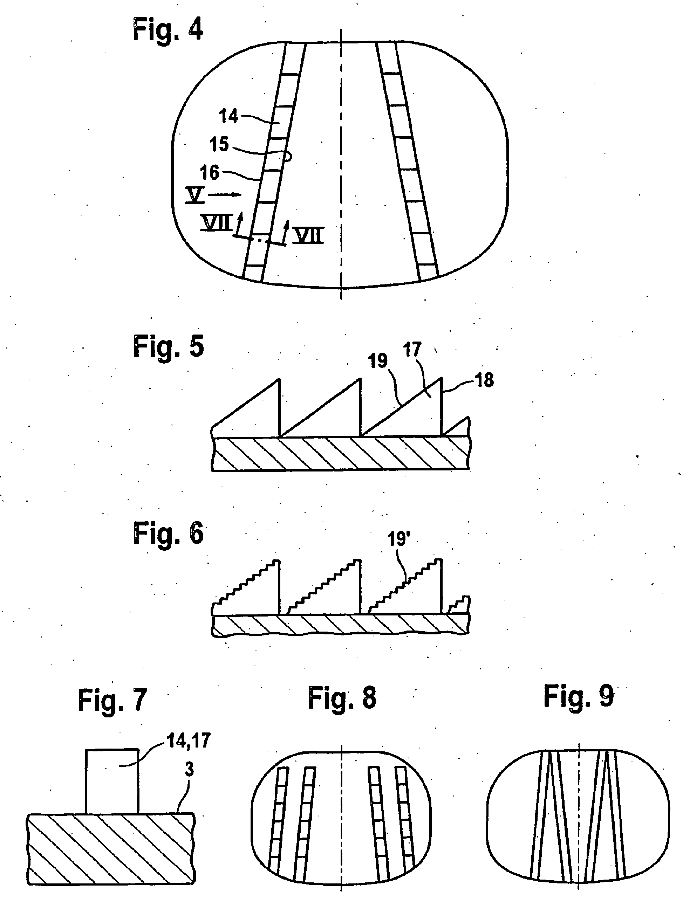

In der Ausführungsform gemäß Fig. 4 ist vorgesehen, daß die Rippen 14, die durch Seitenflächen 15, 16 begrenzt sind, quer gezahnt sind. Die Zähne 17 sind sägezahnförmig ausgebildet mit einer steilen ventralen Fläche 18 und einer als Aufgleitfläche sanft geneigten dorsalen Fläche 19. Das Zahnprofil verläuft zweckmäßigerweise unter 90° zur Medianebene 1, damit die Zähne aller Rippen durch einen einheitlichen Hobelvorgang hergestellt werden können. Wenn dieser Gesichtspunkt nicht maßgebend ist, kann ihre Richtung aber auch anders gewählt werden.

Beispielsweise kann eine parallel zur Implantationsrichtung 2 verlaufende Profilierung verwendet werden, bei der (in Implantationsrichtung gesehen) Lücken zwischen den Zähnen sind. Dies hat den Vorteil, daß der Implantationswiderstand stark vermindert wird. Zwar kann diese Profilierung nicht verhindern, daß die Zähne während der Implantation in gewissem Maße auf die Knochenoberfläche schabend einwirken; aber sie treiben das abgeschabte oder verdrängte Material nicht vor sich her, sondern lassen es in den Lücken der Zähne liegen. Wenn die Zähne ihre endgültige Position erreicht haben, befindet es sich nicht auf ihrer dorsalen Seite, sondern in ihrer unmittelbaren ventralen Nachbarschaft. Es kann daher die postoperative Verankerung der Zähne fördern.

Bei der Ausführung gemäß Fig. 4 kann das Rippenprofil (gesehen in Rippenlängsrichtung) ebenso ausgebildet sein, wie es in Fig. 2 gezeigt ist, d.h. es fällt auf der Seitenfläche 16 sanft ab. Die Aufgleitfläche der Rippe 14 wird dann nicht nur durch die dorsale Fläche 19 jedes einzelnen Zahns 17 sondern auch durch die Seitenfläche 16 der Rippe 14 gebildet. Es kann aber auch ein Rippenprofil gemäß Fig. 7 gewählt werden, bei dem die Seitenfläche 16 ebenso steil ist wie die Seitenfläche 15. In diesem Fall bilden alleine die dorsalen Flächen 19 der Zähne 17 die Aufgleitfläche. Man kann auch Zwischenstufen wählen, bei denen die Seitenfläche 16 unter einem relativ steilen Winkel geneigt ist, was aber in dem für den Implantationsvorgang maßgebenden Schnitt gemäß Fig. 3 einen wesentlich flacheren Aufgleitwinkel bewirkt.

Wenn die dorsale Fläche 19 der Zähne 17 glatt ist (Fig. 5), wirkt sich dies günstig auf die Schonung der Knochenoberfläche während der Implantation aus. Es kann aber auch vorteilhaft sein, sie rauh auszubilden, um zusätzliche Verankerungsmöglichkeit nach der Implantation bereitzustellen. Diese Variante ist in Fig. 6 gezeigt, die auf den Flächen 19' eine feine Zahnung zeigt.

Es ist wichtig, daß das Implantat im dorsalen Bereich des Wirbelzwischenraums gut verankert ist, weil die Möglichkeit besteht, daß die Anschlußplatten sich im ventralen Bereich von den wirbelkörperoberflächen abheben, wenn kurz nach der Implantation, wenn die Anschlußplatten noch nicht mit der Knochenoberfläche verwachsen sind, zufällig eine starke lordotische Biegung der Wirbelsäule erfolgt. Deshalb ist es zweckmäßig, wenn die Rippen 4, 14 bis etwa an den dorsalen Rand der Anschlußfläche geführt sind. Andererseits möchte man die Randbereiche der Wirbelkörperendplatten für die Übertragung der Lastkräfte vom Knochen auf die Prothese nützen, weil sie wegen höherer Dichte zur Kraftaufnahme gut geeignet sind. Die Erfindung wird beiden Wünschen gerecht, wenn die Rippen in mindestens der Hälfte der von ihnen eingenommenen Fläche einen Abstand von mehr als einem Sechstel der AP-Dimension der Anschlußfläche von deren Begrenzung aufweisen. Die AP-Dimension ist die Abmessung in anteroposteriorer Richtung.

Die erfindungsgemäße Anordnung muß sich nicht auf ein Rippenpaar beschränken. Es können vielmehr- wie in Fig 8 und 9 veranschaulicht - mehrere Paare vorhanden sein. Sie sind in den dargestellten Beispielen so angeordnet, daß sie einen sich nach ventral öffnenden v-förmigen Zwischenraum einschließen. Dieser Zwischenraum kann sich aber auch nach dorsal öffnen, wie dies bei der Ausführung gemäß Fig. 9 zwischen den beiden mittleren Rippen der Fall ist.

## Patentansprüche

1. zwischenwirbelprothese, insbesondere für die Halswirbelsäuie, mit zwei gelenkig verbundenen Anschlußplatten, deren für den Anschluß an die benachbarten Wirbelkörper ausgebildeten Anschlußflächen eine an der Wirbelkörperoberfläche anzuliegen bestimmte Grundfläche (3) und davon sich erhebende, selbstschneidende Fixiervorsprünge aufweisen, die von wenigstens einem Paar von Rippen (4, 14) gebildet sind, die gegensinnig schräg zu einer vorbestimmten Implantationsrichtung (2) verlaufen, **dadurch gekennzeichnet, daß** deren mehr entgegen der Implantatiortsrichtung gewendete Seitenflächen (15) steiler sind als eine mehr in Implantationsrichtung gewendete Aufgleitfläche (6, 16).

2. Prothese nach Anspruch 1, **dadurch gekennzeichnet, daß** die Implantationsrichtung (2) die AP-Richtung ist.

3. Prothese nach Anspruch 2, **dadurch gekennzeichnet, daß** die Fixiervorsprünge (4, 14) symmetrisch zur Medianebene (1) angeordnet sind.

4. Prothese nach Anspruch 1, **dadurch gekennzeichnet, daß** die mehr nach ventral gewendete Seitenfläche (5, 15) im wesentlichen lotrecht zur Grundfläche (3) steht.

5. Prothese nach einem der Ansprüche 1 bis 4, **dadurch gekennzeichnet, daß** die Aufgleitfläche (6, 16) mit der Grundfläche (3), gemessen in einer parallel zur Implantationsrichtung (2) und lotrecht zur Grundfläche (3) verlaufenden Ebene, einen Winkel von weniger als 45° einschließt.

6. Prothese nach einem der Ansprüche 1 bis 5, **dadurch gekennzeichnet, daß** die Rippe (4) ein im wesentlichen gleichmäßig durchgehendes Profil aufweist und ihre Aufgleitfläche von ihrer mehr nach dorsal gewendeten Seitenfläche (6) gebildet ist.

7. Prothese nach einem der Ansprüche 1 bis 5, **dadurch gekennzeichnet, daß** die Rippe (14) unter Bildung einer Reihe von spitzen oder scharfen Zähnen (17) mehrfach durchbrochen ist.

8. Prothese nach Anspruch 7, **dadurch gekennzeichnet, daß** die Durchbrechungen in einer parallel zur Grundfläche verlaufenden Schnittebene lotrecht zur Implantationrichtung (2) verlaufen.

9. Prothese nach Anspruch 8, **dadurch gekennzeichnet, daß** die Zähne (17) eine steile ventrale Flanke (18) und eine weniger steile dorsale Flanke (19, 19') haben.

10. Prothese nach Anspruch 9, **dadurch gekennzeichnet, daß** die Zähne (17) ein sägezahnförmiges Profil aufweisen und ihre weniger steilen, dorsalen Flanken (19, 19') eine Aufgleitfläche bilden.

11. Prothese nach Anspruch 7, **dadurch gekennzeichnet, daß** die Durchbrechungen in einer parallel zur Grundfläche verlaufenden Schnittebene parallel zur Implantationsrichtung (2) verlaufen.

12. Prothese nach einem der Ansprüche 1 bis 11, **dadurch gekennzeichnet, daß** der von den Rippen (4, 14) eingenommene Flächenanteil nicht größer als ein Fünftel der Anschlußfläche ist.

13. Prothese nach Anspruch 12, **dadurch gekennzeichnet, daß** die Querschnittsfläche der Rippen (4, 14) in einer in halber Höhe über der Grundfläche (3) verlaufenden Schnittebene nicht größer als ein Zehntel der Anschlußfläche ist.

14. Prothese nach Anspruch einem der Ansprüche 1 bis 13, **dadurch gekennzeichnet, daß** mindestens die Hälfte der von den Rippen (4, 14) eingenommenen Fläche einen Abstand von mehr als einem Sechstel der AP-Dimension der Anschlußfläche von der Begrenzung der Anschlußfläche hat.

15. Prothese nach einem der Ansprüche 1 bis 14, **dadurch gekennzeichnet, daß** die Rippen (4, 14) wenigstens eines Rippenpaars sich nach dorsal einander nähern.

16. Prothese nach Anspruch 15, **dadurch gekennzeichnet**, auf jeder Seite der Symmetrieebene (1) mehrere Rippen parallel zueinander angeordnet sind.

17. Prothese nach einem der Ansprüche 1 bis 16, **dadurch gekennzeichnet, daß** die Höhe der Rippen (4, 14) über der Grundfläche nicht größer als ein Zehntel der AP-Abmessung der Anschlußfläche ist.

18. Prothese nach einem der Ansprüche 1 bis 17, **dadurch gekennzeichnet, daß** der Winkel (alpha), den die mehr nach ventral gewendeten Seitenflächen (5, 15) der Rippen (4, 14) in einer parallel zur Grundfläche verlaufenden Schnittebene miteinander einschließen, kleiner als 60° und vorzugsweise kleiner als 30° ist.

19. Prothese nach Anspruch 18, **dadurch gekennzeichnet, daß** der Winkel (alpha) zwischen 5 und 15° liegt.

20. Prothese nach einem der Ansprüche 1 bis 19, **dadurch gekennzeichnet, daß** die Aufgleitfläche (19') gerauht oder gezahnt ist.

21. Prothese nach einem der Ansprüche 1 bis 20, **dadurch gekennzeichnet, daß** mindestens ein Rippenpaar bis wenigstens auf ein Fünftel der AP-Abmessung der Anschlußfläche an deren dorsale Begrenzung heranreicht.

## Claims

1. An intervertebral prosthesis, in particular for the cervical spine, with two attachment plates which are connected in an articulated manner and whose attachment surfaces are configured for attachment to the adjacent vertebral bodies have a base surface (3) configured to bear on the surface of the vertebral bodies, and self tapping fixing projections rising from said base surface (3), wherein the fixing projections are formed by at least one pair of ribs (4, 14) which extend in opposite directions obliquely with respect to a predetermined implantation direction (2) **characterized in that** its side faces (15) oriented more away from the implantation direction are steeper than a slide-on surface (6, 16) oriented more in the implantation direction.

2. The prosthesis as claimed in claim 1, wherein the implantation direction (2) is the AP direction.

3. The prosthesis as claimed in claim 2, wherein the fixing projections (4, 14) are arranged symmetrically with respect to the median plane (1).

4. The prosthesis as claimed in claim 1, wherein the ventrally oriented side face (5, 15) is substantially perpendicular to the base surface (3).

5. The prosthesis as claimed in claim 1 through 4, wherein the slide-on surface (6, 16) encloses an angle of less than 45° with the base surface (3), measured in a plane extending parallel to the implantation direction (2) and perpendicular to the base surface (3).

6. The prosthesis as claimed in claim 1 through 5, wherein the rib (4) has a substantially uniform and constant profile, and its slide-on surface (6) is formed by its more dorsally oriented side face.

7. The prosthesis as claimed in one of claims 1 through 5, wherein the rib (14) is interrupted a plurality of times in order to form a series of pointed or sharp teeth (17).

8. The prosthesis as claimed in claim 7, wherein the interruptions extend in a sectional plane extending parallel to the base surface and perpendicular to the implantation direction (2).

9. The prosthesis as claimed in claim 8, wherein the teeth (17) have a steep ventral flank (18) and a less steep dorsal flank (19, 19').

10. The prosthesis as claimed in claim 9, wherein the teeth (17) have a sawtooth-shaped profile, and their less steep, dorsal flanks (19, 19') form a slide-on surface.

11. The prosthesis as claimed in claim 7, wherein the interruptions extend in a sectional plane extending parallel to the base surface and parallel to the implantation direction (2).

12. The prosthesis as claimed in claim 1 through 11, wherein the proportion of the surface area taken up by the ribs (4, 14) is not greater than a fifth of the attachment surface.

13. The prosthesis as claimed in claim 12, wherein the cross-sectional surface area of the ribs (4, 14), in a sectional plane extending half way above the base surface (3), is not greater than a tenth of the attachment surface.

14. The prosthesis as claimed in claim 1 through 13, wherein at least half of the surface area taken up by the ribs (4, 14) is at a distance of more than one sixth of the AP dimension of the attachment surface from the boundary of the attachment surface.

15. The prosthesis as claimed in claim 1 through 14, wherein the ribs (4, 14) at least of one rib pair approach one another in the dorsal direction.

16. The prosthesis as claimed in claim 15, wherein several ribs are arranged parallel to one another on each side of the median plane (1).

17. The prosthesis as claimed in claim 1 through 16, wherein the height of the ribs (4, 14) above the base surface is not greater than a tenth of the AP dimension of the attachment surface.

18. The prosthesis as claimed in claim 1 through 17, wherein the angle (alpha), enclosed by the more ventrally oriented side faces (5, 15) of the ribs (4, 14) in a sectional place extending parallel to the base surface, is smaller than 60° and preferably smaller than 30°.

19. The prosthesis as claimed in claim 18, wherein the angle (alpha) lies between 5 and 15°.

20. The prosthesis as claimed in claim 1 through 19, wherein the slide-on surface (19') is roughened or toothed.

21. The prosthesis as claimed claim 1 through 20, wherein at least one rib pair reaches at least as far as a fifth of the AP dimension of the attachment surface to the dorsal boundary thereof.

## Revendications

1. Prothèse intervertébrale, notamment pour les vertèbres cervicales, et présentant deux plaques de raccordement reliées de manière articulée et dont les surfaces de raccordement configurées pour être raccordées au corps de vertèbre voisin présentent une surface de base (3) destinée à venir se placer sur la surface du corps de vertèbre et des saillies de fixation auto-taraudeuses qui en débordent et qui sont formées par au moins une paire de nervures (4, 14) qui s'étendent obliquement en sens opposés en direction d'un dispositif prédéterminé d'implantation (2),
**caractérisée en ce que**
ses surfaces latérales (15) davantage orientées en opposition à la direction d'implantation sont plus raides qu'une surface de glissement (6, 16) orientée dans la direction d'implantation.

2. Prothèse selon la revendication 1, **caractérisée en ce que** la direction d'implantation (2) est la direction antéropostérieure AP.

3. Prothèse selon la revendication 2, **caractérisée en ce que** les saillies de fixation (4, 14) sont disposées symétriquement par rapport au plan médian (1).

4. Prothèse selon la revendication 1, **caractérisée en ce que** la surface latérale (5, 15) davantage orientée dans la direction ventrale est essentiellement perpendiculaire à la surface de base (3).

5. Prothèse selon l'une des revendications 1 à 4, **caractérisée en ce que** dans un plan qui s'étend parallèlement à la direction d'implantation (2) et perpendiculairement à la surface de base (3), la surface de glissement (6, 16) forme un angle inférieur à 45° avec la surface de base (3).

6. Prothèse selon l'une des revendications 1 à 5, **caractérisée en ce que** la nervure (4) présente un profil essentiellement continu et uniforme et **en ce que** sa surface de glissement est formée par ses surfaces latérales (6) orientées davantage en direction dorsale.

7. Prothèse selon l'une des revendications 1 à 5, **caractérisée en ce que** la nervure (14) est interrompue plusieurs fois en formant une série de dents (17) pointues ou tranchantes.

8. Prothèse selon la revendication 7, **caractérisée en ce que** les interruptions s'étendent perpendiculairement à la direction d'implantation (2) dans un plan de coupe qui s'étend parallèlement à la surface de base.

9. Prothèse selon la revendication 8, **caractérisée en ce que** les dents (17) possèdent un flanc ventral raide (18) et un flanc dorsal (19, 19') moins raide.

10. Prothèse selon la revendication 9**, caractérisée en ce que** les dents (17) présentent un profil en dents de scie et **en ce que** leurs flancs dorsaux (19, 19') moins raides forment une surface de glissement.

11. Prothèse selon la revendication 7, **caractérisée en ce que** les interruptions s'étendent parallèlement à la direction d'implantation (2) dans un plan de coupe qui s'étend parallèlement à la surface de base.

12. Prothèse selon l'une des revendications 1 à 11, **caractérisée en ce que** la partie de surface occupée par les nervures (4, 14) n'est pas supérieure à un cinquième de la surface de raccordement.

13. Prothèse selon la revendication 12, **caractérisée en ce que** la surface de la section transversale des nervures (4, 14) dans un plan de coupe qui s'étend à mi-hauteur dans la surface de base (3) n'est pas supérieure à un dixième de la surface de raccordement.

14. Prothèse selon l'une des revendications 1 à 13, **caractérisée en ce qu'**au moins la moitié de la surface occupée par les nervures (4, 14) présente par rapport à la limite de la surface de raccordement une distance de plus du sixième de la dimension antéropostérieure AP de la surface de raccordement.

15. Prothèse selon l'une des revendications 1 à 14, **caractérisée en ce que** les nervures (4, 14) d'au moins une paire de nervures s'approchent l'une de l'autre dorsalement.

16. Prothèse selon la revendication 15, **caractérisée en ce que** plusieurs nervures sont disposées parallèlement les unes aux autres sur chaque côté du plan de symétrie (1).

17. Prothèse selon l'une des revendications 1 à 16, **caractérisée en ce que** la hauteur des nervures (4, 14) au-dessus de la surface de base n'est pas supérieure à un dixième de la dimension antéropostérieure AP de la surface de raccordement.

18. Prothèse selon l'une des revendications 1 à 17, **caractérisée en ce que** dans un plan de coupe qui s'étend parallèlement à la surface de base, l'angle (alpha) que forment les surfaces latérales (5, 15), orientées davantage ventralement, des nervures (4, 14) est inférieur à 60° et de préférence inférieur à 30°.

19. Prothèse selon la revendication 18, **caractérisée en ce que** l'angle (alpha) est compris entre 5 et 15°.

20. Prothèse selon l'une des revendications 1 à 19, **caractérisée en ce que** la surface de glissement (19') est rugueuse ou dentelée.

21. Prothèse selon l'une des revendications 1 à 20, **caractérisée en ce qu'**au moins une paire de nervures s'étend sur au moins un cinquième de la dimension antéropostérieure AP de la surface de raccordement sur sa limite dorsale.
